(19) Europäisches Patentamt
European Patent Office
Office européen des brevets

(11) **EP 4 288 003 B1**

(12) **EUROPÄISCHE PATENTSCHRIFT**

(45) Veröffentlichungstag und Bekanntmachung des Hinweises auf die Patenterteilung:
**29.01.2025 Patentblatt 2025/05**

(21) Anmeldenummer: **21836438.8**

(22) Anmeldetag: **10.12.2021**

(51) Internationale Patentklassifikation (IPC):
***A61F 6/14*** *(2006.01)*

(52) Gemeinsame Patentklassifikation (CPC):
**A61F 6/14**

(86) Internationale Anmeldenummer:
**PCT/EP2021/085119**

(87) Internationale Veröffentlichungsnummer:
**WO 2022/174953 (25.08.2022 Gazette 2022/34)**

(54) **VORRICHTUNG ZUR EMPFÄNGNISVERHÜTUNG UND IONENABGEBENDER KÖRPER**

CONTRACEPTIVE DEVICE AND ION-DELIVERING BODY

DISPOSITIF CONTRACEPTIF ET CORPS ÉMETTEUR D'IONS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **18.02.2021 DE 102021201558**
**18.02.2021 DE 202021100798 U**

(43) Veröffentlichungstag der Anmeldung:
**13.12.2023 Patentblatt 2023/50**

(73) Patentinhaber: **Dühlmeyer, Meira**
**33613 Bielefeld (DE)**

(72) Erfinder: **Dühlmeyer, Meira**
**33613 Bielefeld (DE)**

(74) Vertreter: **Prüfer & Partner mbB**
**Patentanwälte · Rechtsanwälte**
**Sohnckestraße 12**
**81479 München (DE)**

(56) Entgegenhaltungen:
**EP-A1- 0 673 629      EP-A1- 2 308 428**
**US-A- 4 949 732       US-A1- 2009 155 367**
**US-A1- 2017 246 027   US-A1- 2018 235 803**
**US-A1- 2020 054 481**

**Beschreibung**

Die Erfindung betrifft einen ionenabgebenden Körper für eine Vorrichtung zur Empfängnisverhütung sowie eine solche Vorrichtung.

Zur Empfängnisverhütung sind Intrauterinpessare, kurz IUP, bekannt, die eine hormonfreie Empfängnisverhütung ermöglichen. Ein vielfach eingesetztes Intrauterinpessar ist dabei die sogenannte klassische Kupferspirale, die aus einem Kunststoffrahmen besteht, der in Anteilen mit Kupferdraht umwickelt ist. Diese wird zur Empfängnisverhütung (Kontrazeption) in die Gebärmutter (Uterus) eingesetzt. Durch die Abgabe von Kupferionen soll sich die Gebärmutterschleimhaut und der Schleim am Muttermund verändern und die Beweglichkeit und Lebensdauer von Spermien herabgesetzt werden, was letztlich neben anderen diskutierten Wirkprinzipien zur Empfängnisverhütung führt. Alternativ, nicht hormonfrei, gibt es IUP's, die Hormone abgeben und über den klassischen Weg der Unterdrückung der Hypophyse eine antikonzeptive Wirkung ausüben

Eine weitere Variante eines Intrauterinpessars stellt die sogenannte Kupferkette dar. Diese Variante der Cu-IUP (Kupferspirale) besteht aus einem Kunststofffaden mit darauf aufgefädelten Kupferröhrchen. In Abhängigkeit der Größe der Gebärmutter werden üblicherweise Kupferketten mit vier oder sechs solcher Körper eingesetzt. Ein einführungsseitiges Ende des Kunststofffaden der Kupferkette weist einen kleinen Knoten auf, der zur Verankerung der Kupferkette in den Gebärmuttermuskel eingeschoben wird. Die Kupferkette basiert auf dem gleichen Wirkprinzip wie die Kupferspirale, kann sich aufgrund ihrer vergleichsweise kleinen und flexiblen Ausgestaltung aber besser an die Gebärmutter anpassen. Kupferketten-IUP weisen gegenüber herkömmlichen Kupfer-IUPs den Vorteil auf, dass ihre Anwendung unter anderem auch bei Nullipara möglich ist, da es zu keiner Verstärkung der Dysmenorrhoe kommt. Zudem tritt selten die Folge einer Hypermenorrhoe auf, wie bei einem herkömmlichen Kupfer-IUP. Der Pearl-Index ist deutlich niedriger. Auch können längere Liegedauern erreicht werden, da keine Kunststoffanteile erforderlich sind, die verspröden und damit einhergehend nach durchschnittlich 3 bis 4 Jahren zerbrechen können. Der Verzicht auf einen Kunststoffrahmen bedingt darüber hinaus ein geringeres Infektionsrisiko. Aufgrund der fehlenden Tubenreizung durch Kunststoffärmchen kann die Quote an Extrauteringraviditäten verringert werden. Gemäß ihrem flexiblen Aufbau werden Kupferketten häufig auch als rahmenlose Intrauterinpessare bezeichnet.

In diesem Zusammenhang betrifft die US 2009/155367 A1 ein Verfahren zur Herbeiführung einer Eileiterblockade als Mittel zur Empfängnisverhütung bei Frauen. Das Verfahren umfasst den Kontakt des inneren Oberflächengewebes eines Eileiters mit einem Silbernitrat enthaltenden Substrat und die Abgabe einer ausreichenden Menge Silbernitrat an das Gewebe. In einer Ausführungsform wird mindestens ein silbernitrathaltiges Kügelchen durch die Gebärmutteröffnung des Eileiters unter Verwendung eines Katheters oder einer anderen zur Manipulation des Kügelchens geeigneten Vorrichtung eingeführt.

Die US 2018/235803 A1 betrifft ein Intrauterinpessar (IUP) mit ovaler Form, wobei die Vorrichtung einen Kern aus magnetischem Material, ein inertes Material oder eine Kupferbeschichtung des Kerns umfasst, wobei die Beschichtung einen pharmazeutischen Wirkstoff, Kupfer oder eine Kombination umfasst davon.

Die US 2017/246027 A1 betrifft ein Intrauterinpessar. Das Intrauterinpessar umfasst einen Draht mit einem Abschnitt, der eine dreidimensionale (3D) Struktur bilden kann. Die 3D-Struktur ist durch eine Druckkraft, die größer ist als die durch eine entspannte Gebärmutterhöhle auf sie ausgeübte Kraft, elastisch zu einer teilweise kollabierten Konfiguration verformbar. Die dreidimensionale Struktur ist auch in der Lage, sich als Reaktion auf die Kontraktion und Expansion der Gebärmutterhöhle elastisch zusammenzuziehen und auszudehnen.

Die EP 0 673 629 A1 betrifft ein intrauterines Kontrazeptivum mit mindestens zwei flexiblen Armen. Das Kontrazeptivum ist mit mindestens zwei nahezu horizontalen Armen mit festen Spitzen ausgestattet. Die Arme sind vorne und hinten mit Aussparungen in Längsrichtung versehen. Der zentrale Punkt des Kontrazeptivums ist mit einem flexiblen Faden versehen, der von dem als Kontrazeptivum wirkenden Mittel umgeben ist. Die Wirkmittel können aus Kupferringen bestehen, die von einem unterhalb des Gewindes befindlichen Haltemittel gehalten werden. Neben Kupferringen kann ein flexibler Schlauch mit Öffnungen verwendet werden, in den ein wirksames Verhütungsmittel, beispielsweise ein Hormonpräparat, eingeführt wird.

Die EP 2 308 428 A1 betrifft ein Verhütungsmittel, das sowohl in der Gebärmutter als auch in den Eileitern wirksam ist und mindestens ein metabolisch aktives Element umfasst, wobei die Vorrichtung so angeordnet ist, dass sie eine längliche, nicht starre Struktur mit begrenzten Abmessungen bildet, die mit der Gebärmutterhöhle kompatibel ist.

Die US 4 949 732 A betrifft ein Einhandgerät zum Einführen und Fixieren eines IUP, das über ein Penetrationselement mit einer Nadel am distalen Ende zum Halten eines Befestigungselements eines IUP verfügt.

Trotz der kleinen und flexiblen Ausgestaltung der Kupferkette konnte bei Frauen, denen diese eingesetzt wurde, sogenannte zyklusunabhängige IUP-bedingte Schmier- oder Zwischenblutungen festgestellt werden. Als mögliche Ursache hierfür konnte mittels Hysteroskopie in über 300 Fällen als Ursache Mikroerosionen am Endometrium über die scharfkantigen Enden der Kupferröhrchen der Kupferkette eruiert werden (also Verletzungen endometrialer Gefäße).

In Anbetracht der vorstehenden Ausführung liegt der vorliegenden Erfindung die Aufgabe zugrunde, ein rahmenloses Intrauterinpessar bereitzustellen, das Erosionen am Endometrium reduzieren oder sogar verhindern kann.

Die Aufgabe wird durch eine Vorrichtung zur Empfängnisverhütung nach Anspruch 2 und einen ionenabgebender Körper für eine solche Vorrichtung nach Anspruch 1 gelöst. Vorteilhafte Weiterbildungen der Erfindung sind in den abhängigen Ansprüchen enthalten.

Erfindungsgemäß weist die Vorrichtung zur Empfängnisverhütung zumindest einen Faden sowie zumindest einen ionenabgebenden Körper mit zumindest einer Durchgangsbohrung mit zumindest zwei Durchgangsbohrungsöffnungen, durch die der zumindest eine Faden verläuft, auf, wobei eine sich von einer der Durchgangsbohrungsöffnungen zur anderen der Durchgangsbohrungsöffnungen erstreckende Außenfläche des ionenabgebenden Körpers keine nach außen weisende Kante aufweist.

Durch den Wegfall nach außen weisender Kanten, wie sie beispielsweise bei der Verwendung von Kupferröhrchen an den Stirnflächen im Übergang zu den äußeren Seitenflächen auftreten würden, wird das Risiko von Erosionen am Endometrium minimiert. Die stärksten Erosionen waren zudem durch die Kanten zu beobachten, die durch die Befestigung mittels Faltpressung auf den Faden entstehen.

Auch wenn der ionenabgebender Körper hier beispielsweise immer noch eine Kante am Übergang der Außenfläche zur Durchgangsbohrung aufweisen kann, weist diese Kante jedoch nicht nach außen, sondern bildet lediglich eine Flächenaussparung durch die jeweilige Durchgangsbohrungsöffnung. Der Übergang von der Außenfläche zur Durchgangsbohrung kann zudem auch über einen Radius ausgebildet werden. Ein entscheidender Vorteil gegenüber den Kupferröhrchen ist auch das Entfallen der scharfkantigen Enden durch die Befestigungspressung. Die hier vorliegende Erfindung bietet über die Durchgangsbohrungen mit dem Versenken der Knotenbefestigung ins Körperinnere einen totalen Entfall der scharfkantigen Enden.

Als Faden kann beispielsweise ein Faden aus Polypropylen verwendet werden. Der Begriff Faden umfasst biegeschlaffe Gebilde mit einer dominierenden eindimensionalen Erstreckung und ist nicht nur auf aus Kunststoff, Fasern oder anderweitigen Materialien gebildete Fäden gerichtet, sondern kann auch durch anderweitige Schnüre oder Ketten in fadenähnlicher Ausprägung ausgebildet sein. Zudem können auch Kombinationen mehrere solcher Fäden, beispielsweise zur Verstärkung, eingesetzt werden. Zur Vereinfachung wird im Folgenden der Begriff Faden verwendet, wobei die Offenbarung hierzu auch gleichermaßen auf mehrere Fäden übertragbar und somit anwendbar ist, sofern sich dies im Kontext nicht ausschließt. Die ionenabgebenden Körper können dabei über Knoten, deren Durchmesser größer als der minimale Durchmesser der Durchgangsbohrung des jeweiligen ionenabgebenden Körper ist, zumindest in einer Erstreckungsrichtung des Fadens in ihrer Möglichkeit, sich relativ zum Faden zu bewegen, beschränkt werden. Ein Ende des Fadens weist einen Verankerungsknoten als Vorrichtungsfixierung auf, der beim Einsetzen der Vorrichtung in eine Gebärmutter in den Gebärmuttermuskel eingeschoben werden kann, um hierüber die Vorrichtung in der Gebärmutter zu halten. Unterhalb des Verankerungsknotens bzw. zwischen Verankerungsknoten und dem zumindest einen ionenabgebenden Körper kann zudem ein Metallclip gesetzt werden, um eine sonografisch detektierbare Referenz auszubilden. Beispielsweise kann hierzu ein Edelstahlclip verwendet werden. Der Metallclip kann aber auch aus einem anderen Metall oder einer entsprechenden Legierung gebildet werden, die ergänzend oder alternativ einer sonografischen Detektierbarkeit auch eine bakterien- und/oder pilzhemmende Wirkung aufweist, um entsprechenden Einflüssen durch das Einsetzen der Vorrichtung in den Gebärmuttermuskel entgegenwirken zu können.

Zudem weist die Durchgangsbohrung zumindest zwei unterschiedliche Durchmesser auf.

Durch zumindest zwei unterschiedliche Durchmesser der Durchgangsbohrungen kann beispielsweise in einem Abschnitt der Durchgangsbohrung mit größerem Durchmesser eine größere Oberfläche bereitgestellt werden, während der ionenabgebende Körper zumindest durch den Abschnitt der Durchgangsbohrung mit dem kleineren Durchmesser mit dem Knoten im Faden zur Beschränkung der Relativbewegung des ionenabgebenden Körpers zusammenwirken kann. Sofern der größere Durchmesser größer und der kleinere Durchmesser kleiner als der Knoten ist, kann der Knoten über den Abschnitt der Durchgangsbohrung mit dem größeren Durchmesser abgedeckt werden, so dass dieser keine Störkontur bildet oder auch der Abstand mehrerer ionenabgebenden Körper der Vorrichtung verringert werden kann. Die Durchgangsbohrung kann auch mehr als zwei Abschnitte mit unterschiedlichen Durchmessern aufweisen, um beispielsweise in Bezug auf unterschiedliche Knotengrößen und/oder vorzusehende Abstände mehrerer ionenabgebender Körper flexible Haltepunkte bieten zu können. In Anbetracht der vorstehenden Beispiele sind die unterschiedlichen Durchmesser insbesondere so auszugestalten, dass sie sich in einer Richtung verkleinern, hierbei bevorzugt in Richtung des Verankerungsknotens, oder vergrößern.

In einer Ausgestaltung ist die Außenfläche des ionenabgebenden Körpers zumindest abschnittsweise als Flächenabschnitt eines Ellipsoids mit a, b und c als Längen der Halbachsen eines solchen Ellipsoids, insbesondere in kartesischen Koordinaten über die Gleichung

$$\frac{x^2}{a^2} + \frac{y^2}{b^2} + \frac{z^2}{c^2} = 1$$

beschreibbar, wobei a, b und c größer null sind.

Die Außenfläche des ionenabgebenden Körpers wird somit zumindest abschnittsweise über Flächenabschnitte eines Ellipsoids gebildet. Insbesondere weist die Grundform des ionenabgebenden Körpers insgesamt im Wesentlichen die Form eines Ellipsoids auf. Der Begriff im Wesentlichen bezieht sich dabei auf eine erkennbare Grundform, wobei je nach konkreter Ausgestaltung einzelne Flächenbereiche des ionenabgebenden Körpers rückversetzt und/oder vorstehend ausgebildet sein können, um beispielsweise die Oberfläche zur Abgabe von Ionen zu vergrößern. Zum Beispiel kann die Oberfläche konkave nach innen gerichtete Ausbuchtungen, ähnlich der Oberflächenstruktur eines Golfballs, als rückversetzte Flächenbereiche aufweisen. Analog hierzu besteht auch die Möglichkeit konvexer nach außen gerichteter Ausbuchtungen, wobei vorstehende Flächenbereiche gemäß dem Grundgedanken der Erfindung keine Kanten oder Spitzen aufweisen, die das Risiko von Erosionen mit sich bringen. Die hier beispielhaft angeführten Ausbuchtungen führen jedoch nicht zu einer Änderung der erkennbaren Grundform des ionenabgebenden Körpers als Ellipsoid. Auch die durch die Durchgangsbohrungsöffnungen der Durchgangsbohrung gebildeten Aussparungen in der Außenfläche des ionengebenden Körpers führen nicht zu einer wesentlichen Änderung der erkennbaren Grundform.

Sofern die Außenfläche des ionenabgebenden Körpers in der erkennbaren Grundform lediglich abschnittsweise durch Flächenabschnitte eines Ellipsoids gebildet wird, so ist auch hier die übrige Außenfläche so zu gestalten, dass die Flächenübergänge rund ausgeführt werden.

Hinsichtlich der Beschreibung der Außenfläche oder von Flächenabschnitten der Außenfläche über einen Ellipsoid stellen die Längen der Halbachsen a, b und c die jeweiligen Längen in drei zueinander senkrechten Achsen dar. In einem kartesischen Koordinatensystem entspricht beispielsweise die Länge der Halbachse a der Länge der Halbachse in x-Richtung, die Länge der Halbachse b der Länge der Halbachse in y-Richtung und die Länge der Halbachse c der Länge der Halbachse in z-Richtung.

Insbesondere erstreckt sich eine Längsachse des ionenabgebenden Körpers im Wesentlichen parallel zu einer Erstreckungsrichtung der Durchgangsbohrung des ionenabgebenden Körpers und die Längen der Halbachsen a, b erstrecken sich senkrecht zur Längsachse und die Länge der Halbachse c erstreckt sich in Richtung der Längsachse.

Demnach korrespondiert die Richtung der Längsachse des ionenabgebenden Körpers im Wesentlichen zur Erstreckungsrichtung der Durchgangsbohrung und ist insbesondere koaxial hierzu. Der Begriff im Wesentlichen in Bezug auf die Erstreckungsrichtung bezieht sich darauf, dass die Durchgangsbohrung nicht zwingend geradlinig sein muss, eine Erstreckungsrichtung dennoch als Verbindung der eingangsseitigen und ausgangsseitigen Durchgangsbohrungsöffnungen angegeben werden kann.

Gemäß einer Ausgestaltung sind die Längen der Halbachsen a, b und c gleich.

Somit bildet die Außenfläche des ionenabgebenden Körpers insgesamt oder zumindest abschnittsweise eine Kugel als eine Form eines Ellipsoids mit gleich langen Halbachsen a, b und c aus.

Insbesondere liegen die Längen der Halbachsen a, b und c zwischen 1 mm und 5,5 mm, insbesondere zwischen 1,5 mm und 5 mm.

Über größere Längen der Halbachsen können gerade im Hinblick auf die Außenfläche des ionenabgebenden Körpers insgesamt als Kugel größere Kugeldurchmesser und somit größere Flächen zur Ionenabgabe vorgesehen werden. Andererseits können beispielsweise kleinere Kugeln als ionenabgebender Körper flexibler eingesetzt werden.

Alternativ stimmen zwei der Längen der Halbachsen a, b und c überein und eine der Längen der Halbachsen a, b und c weicht hiervon ab.

Die Außenfläche des ionenabgebenden Körpers bildet somit in ihrer Grundform ein Rotationsellipsoid aus. Alternativ kann die Außenfläche auch lediglich zumindest einen Flächenabschnitt aufweisen, der über einen entsprechenden Flächenausschnitt eines Ellipsoids gebildet wird.

Sofern die Außenfläche des ionenabgebenden Körpers in ihrer Grundform über einen Rotationsellipsoid gebildet wird, kann hierüber beispielsweise im Vergleich zu einer Kugel bei gleichem Volumen eine größere Oberfläche zur Ionenabgabe bereitgestellt werden.

In einer Ausgestaltung sind die Längen der Halbachsen a und b gleich.

Beispielsweise erstreckt sich die Länge der Halbachse c parallel, insbesondere koaxial zur Längsachse des ionenabgebenden Körpers. Die Längsachse des ionenabgebenden Körpers ist dabei im Wesentlichen parallel zur Erstreckungsrichtung der Durchgangsbohrung des ionenabgebenden Körpers. Demnach ergibt sich bei gleichen Längen der Halbachsen a und b und einer hierzu unterschiedlichen Länge der Halbachse c ein rotationssymmetrisches Ellipsoid mit der Längsachse des ionenabgebenden Körpers als Rotationsachse für die Symmetrie. Ein Verkippen des ionenabgebenden Körpers um eine Achse senkrecht zur Längsachse ist dabei zumindest bei koaxialer Lage der Längsachse in Bezug auf die Erstreckungsrichtung der Durchgangsbohrung weniger wahrscheinlich.

Insbesondere liegen die Längen der Halbachsen a und b zwischen 1 mm und 2 mm, insbesondere bei etwa 1,5 mm, und die Länge der Halbachse c liegt zwischen 1,5 mm und 5,5 mm, insbesondere zwischen 2 mm und 5 mm.

Hieraus ergeben sich für einen ionenabgebenden Körper als Rotationsellipsoid mit seiner Längsachse als Rotationsachse Abmessungen von 2 mm bis 4 mm, insbesondere 3 mm, für einen Kreisdurchmesser senkrecht zur Längsachse, und 3 mm bis 11 mm, insbesondere 4 bis 10 mm, für die Länge in Richtung der Längsachse. Insbesondere bei größerer Länge in Richtung der Längsachse gegenüber dem Kreisdurchmesser, kann bei gleicher Oberfläche der Kreisdurchmesser verringert werden, um eine Einführung der Vorrichtung zum Einsetzen in die Gebärmutter zu erleichtern bzw. zumindest nicht zu behindern.

Alternativ zur Ausgestaltung gleich langer Halbachsen a und b sind die Längen der Halbachsen a und c oder b und c gleich.

Hierüber kann der ionenabgebende Körper ein Rotationsellipsoid mit einer Rotationsachse senkrecht zur vorstehend beschriebenen Längsachse ausbilden.

Insbesondere liegt die Länge der Halbachse c zwischen 1 mm und 5,5 mm, insbesondere zwischen 1,5 mm und 5 mm, und die hiervon unterschiedliche Länge der Halbachse a oder b liegt zwischen 1 mm und 3,5 mm, insbesondere zwischen 1,5 mm und 3 mm.

Gerade bei einer kürzeren Länge der Halbachse c gegenüber der hiervon abweichenden Länge der Halbachse a oder b kann sich der ionenabgebende Körper bei einer Einführung der Vorrichtung zum Einsetzen in die Gebärmutter leichter in Einführungsrichtung verkippen, so dass trotz einseitiger größerer Abmessung eine Behinderung hierdurch während des Einführvorgangs vermieden werden kann.

Gemäß einer weiteren Ausgestaltung sind die Längen der Halbachsen a, b und c jeweils zueinander unterschiedlich.

Der ionenabgebende Körper kann hierdurch beispielsweise als triaxiales bzw. dreiachsiges Ellipsoid ausgebildet bzw. beschrieben werden. Hier, wie aber auch bei allen anderen Ausgestaltungen, bei denen die Außenfläche des ionenabgebenden Körpers in ihrer Grundform über einen Ellipsoid beschreibbar ist, ergeben sich trotz individueller Ausgestaltungsvarianten, wie im Hinblick auf Abmessungen und/oder Oberflächenstrukturen, immer noch vergleichsweise einfache Fertigungsvorgaben.

Gemäß einer Ausgestaltung enthält der ionenabgebende Körper ein Metall, insbesondere Kupfer, eine Kupferlegierung, Gold, eine Goldlegierung oder eine Kupfer-Gold-Legierung, oder wird hieraus gebildet.

Beispielsweise kann durch Kupferionen, die bei Verwendung von Kupfer oder einer Kupfer enthaltenden Legierung freigesetzt werden, eine toxische und hemmende Wirkung auf Spermien herbeigeführt werden, die zu einer Herabsetzung der Beweglichkeit und Lebensdauer der Spermien führen kann. Zudem kann die Verwendung von Gold oder einer Gold-Kupfer-Legierung bzw. Kupfer-Gold-Legierung eine bakterien- und pilzhemmende Wirkung aufweisen, die das Risiko für Infektionen und Entzündungen verringern kann. Ebenso kann ein mikrogalvanischer Effekt bei einer Gold-Kupfer-Legierung bzw. Kupfer-Gold-Legierung positive Auswirkungen auf die Empfängnisverhütung haben.

Die vorgenannten Metalle und/oder deren Legierungen können den ionenabgebenden Körper vollständig oder lediglich teilweise ausbilden. Beispielsweise kann auch nur ein Grundkörper des ionenabgebenden Körpers, der beispielsweise aus Kostengründen aus Kunststoff besteht, mit einer Beschichtung mit einem der vorgenannten Metalle und/oder deren Legierungen versehen werden. Die Beschichtung kann auch auf eine Fläche beschränkt werden, die für eine ausreichende Ionenabgabe geeignet ist. Die Beschichtung liegt dabei zur Ionenabgabe nach außen hin frei.

Vorteilhafterweise weist die Vorrichtung 2 bis 5, insbesondere 3 oder 4, ionenabgebende Körper auf, durch deren jeweilige Durchgangsbohrungen der zumindest eine Faden verläuft.

Die Anzahl der ionenabgebenden Körper wird dabei auf die insgesamt abzugebende Ionenmenge sowie zur Aufnahme geeignete Abmessungen und Geometrieausgestaltungen der ionenabgebenden Körper abgestimmt. Die von dem Faden durchlaufenden ionenabgebenden Körper können in ihren Abmessungen, ihren Geometrieausgestaltungen und/oder ihrer Materialauswahl gleich und/oder unterschiedlich sein. Bei zumindest teilweise unterschiedlichen ionenabgebenden Körpern, also zumindest zwei in einem Ausprägungsmerkmal unterschiedlichen ionenabgebenden Körpern, können diese flexibel gemäß der vorzusehenden Anwendung zusammengestellt werden.

Die Erfindung betrifft einen ionenabgebender Körper für eine Vorrichtung zur Empfängnisverhütung gemäß der vorstehenden Beschreibung. Mögliche Ausgestaltungen und damit verbundene Vorteile der jeweiligen ionenabgebenden Körper ergeben sich analog zu den vorstehenden Ausführungen.

Im Folgenden wird die Erfindung unter Bezugnahme auf die beigefügten Figuren näher erläutert. Die Figuren zeigen im Einzelnen:

Figur 1 eine schematische Querschnittsansicht entlang einer Längsachse einer Vorrichtung zur Empfängnisverhütung gemäß einer ersten Ausführungsform einer erfindungsgemäßen Vorrichtung;

Figur 2 eine schematische Ansicht eines ionenabgebenden Körpers zur Darstellung der Beschreibung als Ellipsoid;

Figur 3a eine schematische Querschnittsansicht einer exemplarischen ersten Ausführungsform eines ionenabgebenden Körpers entlang der Längsachse der Durchgangsbohrung;

Figur 3b eine Seitenansicht auf den ionenabgebenden Körper gemäß Figur 3a;

Figur 3c eine Ansicht von oben auf den ionenabgebenden Körper gemäß den Figuren 3a und 3b;

Figur 3d eine Ansicht von unten auf den ionenabgebenden Körper gemäß den Figuren 3a, 3b und 3c;

Figur 4a eine schematische Querschnittsansicht einer exemplarischen zweiten Ausführungsform eines ionenabgebenden Körpers entlang der Längsachse der Durchgangsbohrung;

Figur 4b eine Seitenansicht auf den ionenabgebenden Körper gemäß Figur 4a;

Figur 4c eine Ansicht von oben auf den ionenabgebenden Körper gemäß den Figuren 4a und 4b;

Figur 4d eine Ansicht von unten auf den ionenabgebenden Körper gemäß den Figuren 4a, 4b und 4c;

Figur 5a eine schematische Querschnittsansicht einer exemplarischen dritten Ausführungsform eines ionenabgebenden Körpers entlang der Längsachse der Durchgangsbohrung;

Figur 5b eine Seitenansicht auf den ionenabgebenden Körper gemäß Figur 5a;

Figur 5c eine Ansicht von oben auf den ionenabgebenden Körper gemäß den Figuren 5a und 5b;

Figur 5d eine Ansicht von unten auf den ionenabgebenden Körper gemäß den Figuren 5a, 5b und 5c;

Figur 6a eine schematische Querschnittsansicht einer exemplarischen vierten Ausführungsform eines ionenabgebenden Körpers entlang der Längsachse der Durchgangsbohrung;

Figur 6b eine Seitenansicht auf den ionenabgebenden Körper gemäß Figur 6a;

Figur 6c eine Ansicht von oben auf den ionenabgebenden Körper gemäß den Figuren 6a und 6b; und

Figur 6d eine Ansicht von unten auf den ionenabgebenden Körper gemäß den Figuren 6a, 6b und 6c.

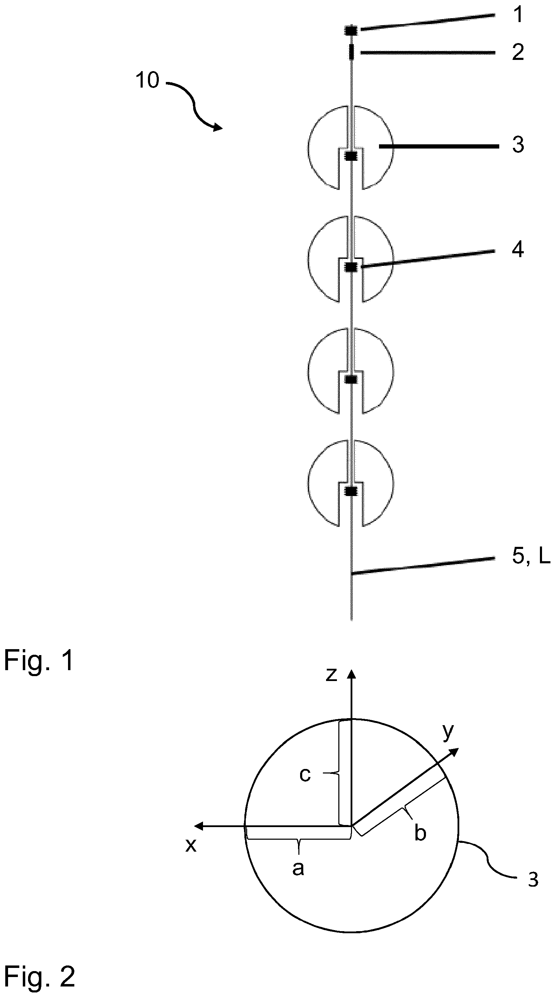

Die Begriffe zu den Ansichtsbezügen, wie "von oben", "von unten" oder Seitenansicht, beziehen sich auf die Zeichnungsansicht gemäß Figur 1 bzw. den Figuren 3a, 4a, 5a und 6a. Im übertragenen Sinne bezieht sich der Begriff "von oben" auf einen Bereich eines ionenabgebenden Körpers, der in Erstreckungsrichtung des durch ihn verlaufenden Fadens in eine Einführungsrichtung einer Vorrichtung zur Empfängnisverhütung weist. Die anderen Ansichtsangaben leiten sich hiervon analog ab.

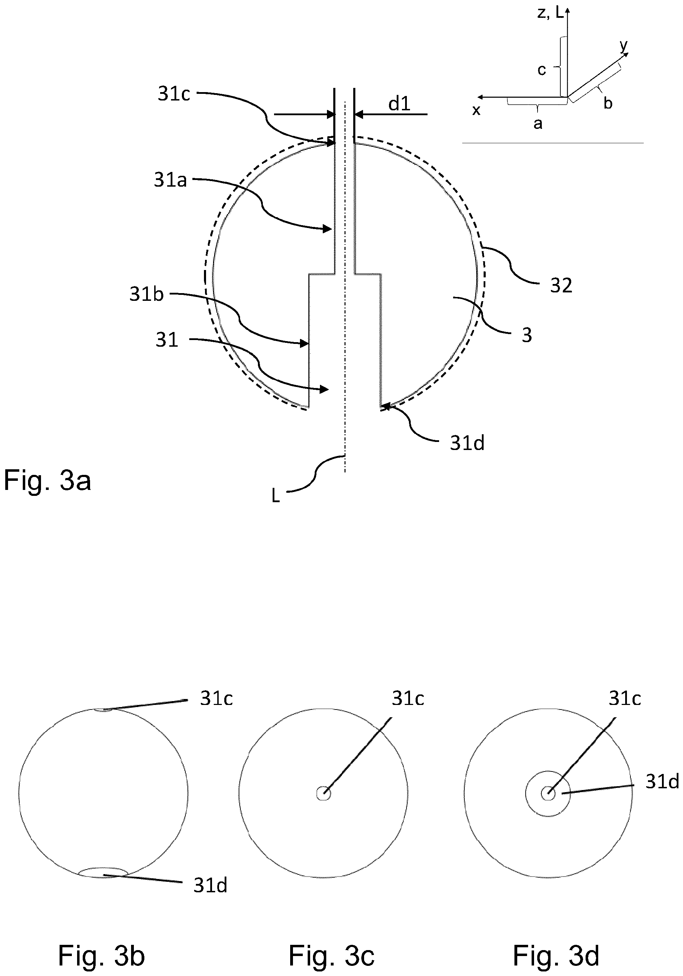

Figur 1 zeigt eine schematische Querschnittsansicht entlang einer Längsachse L einer Vorrichtung 10 zur Empfängnisverhütung gemäß einer ersten Ausführungsform einer erfindungsgemäßen Vorrichtung. Die Vorrichtung 10 umfasst vier ionenabgebende Körper 3 sowie einen Faden 5, der durch Durchgangsbohrungen 31 (Figuren 3a, 4a, 5a, 6a) der ionenabgebenden Körper 3 verläuft. Die Längsachse L bezieht sich auf eine Erstreckungsrichtung der Durchgangsbohrungen 31 der ionenabgebenden Körper 3, die hier zur Erstreckungsrichtung des Fadens 5 durch die ionenabgebenden Körper 3 korrespondiert. Auch wenn in der vorliegenden Ausführungsform vier ionenabgebende Körper 3 gezeigt sind, kann die Anzahl der ionenabgebenden Körper auch hiervon abweichen. Die Anzahl der ionenabgebenden Körper 3 kann gemäß der vorzusehenden Wirkungseffekte und damit verbundenen Auswahl der Abmessungen, Geometrieformen und/oder Materialien auch geringer oder größer ausfallen.

Der Faden 5 weist vier Knoten 4 auf, die als Abstandshalter der ionenabgebenden Körper fungieren. Hierzu sind die Knoten 4 derart dimensioniert, dass sie größer als ein minimaler Durchmesser der Durchgangsbohrungen sind. Demnach können sich die ionenabgebenden Körper 3 zwischen zweien der Knoten 4 jeweils lediglich in dem dadurch vorgegebenen Abstand relativ zum Faden 5 bewegen. Alternativ zur Verwendung der Knoten 4 können aber auch anderweitige Fadenverdickungen oder an dem Faden 5 angeordnete Clips genutzt werden, die in ihren Abmessungen eine entsprechende Funktion ermöglichen. Zudem weist der Faden 5 an einem einführungsseitigen Ende zum Einsetzen der Vorrichtung 10 in eine Gebärmutter einen Verankerungsknoten 1 auf. Der Verankerungsknoten 1 wird zur Fixierung der Vorrichtung 10 in einer Gebärmutter in den Gebärmuttermuskel hineingeschoben und dort gehalten. Zudem ist der Verankerungsknoten 1 ebenfalls derart dimensioniert, dass der dem Verankerungsknoten 1 zugewandte ionenabgebende Körper 3 hierdurch gleichermaßen in seiner Relativbewegung beschränkt wird und somit auf dem Faden 5 gehalten wird. Somit sind alle ionenabgebende Körper 3 jeweils zwischen zwei Knoten 4 oder einem Knoten 4 und dem Verankerungsknoten 1 angeordnet und weisen ein dadurch vorgegebenes Bewegungsspiel auf. Alternativ können die Knoten 4 und/oder der Verankerungsknoten 1 auch derart voneinander beabstandet sein, dass zumindest einer der ionenabgebenden Körper 3 kein Bewegungsspiel aufweist und daher in fester Positionsbeziehung zum Faden 5 gehalten wird. In der hier gezeigten Ausführungsform weist der Faden 5 zudem einen Edelstahlclip 2 auf, der zwischen dem Verankerungsknoten 1 und dem dem Verankerungsknoten 1 zugewandten ionenabgebenden Körper 3 angeordnet ist. Dieser dient der sonografischen Detektion und kann beispielsweise bei der Einführung bzw. dem Einsetzen der Vorrichtung 10 in eine Gebärmutter als Orientierungshilfe erfasst werden. Entsprechend befindet sich der Edelstahlclip 2 insbesondere in der Nähe des Verankerungsknotens 1. In einer Ausführungsvariante kann der Edelstahlclip 2 auch derart dimensioniert sein, dass dieser anstelle des Verankerungsknotens 1 die Relativbewegung des dem Verankerungsknoten 1 zugewandten Körpers 3 in Richtung des Verankerungsknotens 1 beschränkt.

Figur 2 zeigt eine schematische Ansicht des ionenabgebenden Körpers 3 zur Darstellung der Beschreibung als Ellipsoid. Der ionenabgebende Körper 3 gemäß Figur 2 ist eine Kugel als spezifische Ausführungsvariante eines Ellipsoids. Aus Gründen der Übersichtlichkeit ist die Durchgangsbohrung 31 in dieser Ansicht nicht gezeigt. Zudem hat die Durchgangsbohrung auch keinen Einfluss auf die durch die Außenflächen des ionenabgebenden Körper 3 ausgebildete erkennbare Grundform als Ellipsoid, hier konkret als Kugel.

Anhand des in Figur 2 dargestellten ionenabgebenden Körpers 3 wird die Beschreibung eines Ellipsoids unter Verwendung kartesischer Koordinaten exemplarisch erläutert. So wie hierüber konkret der ionenabgebende Körper 3 als Kugel beschreibbar ist, können alternativ auch weitere von einer Kugel abweichende Ellipsoide oder entsprechende Flächenabschnitte eines ionenabgebenden Körpers beschrieben werden.

Die Beschreibung eines Ellipsoids erfolgt hier über die Länge seiner Halbachsen a, b und c, deren Betrag jeweils größer null ist. In kartesischen Koordinaten ist der Ellipsoid dann über die Gleichung

$$\frac{x^2}{a^2} + \frac{y^2}{b^2} + \frac{z^2}{c^2} = 1$$

darstellbar. Die Länge der Halbachse a entspricht dabei einer Länge der Halbachse in x-Richtung, die Länge der Halbachse b einer Länge der Halbachse in y-Richtung und die Länge der Halbachse c einer Länge der Halbachse in z-Richtung, wobei die Richtungsachsen jeweils senkrecht zueinander sind. In den gezeigten Ausführungsformen weist die z-Richtung jeweils in Richtung der Längsachse L. Grundsätzlich kann das Koordinatensystem aber auch hiervon abweichend ausgerichtet sein. Gemäß der in Figur 2 gezeigten Kugel als Grundform des ionenabgebenden Körpers 3 sind hier die Längen der Halbachsen a, b und c gleich.

Figur 3a zeigt eine schematische Querschnittsansicht einer exemplarischen ersten Ausführungsform eines ionenabgebenden Körpers 3 entlang der Längsachse L der Durchgangsbohrung 31. Wie vorstehend beschrieben, ist der ionenabgebende Körper 3 in der ersten Ausführungsform in seiner Grundform eine Kugel. Mit anderen Worten bildet die Außenfläche 32 des ionenabgebenden Körpers 3 im Wesentlichen eine Kugel. Die Kugelform der Außenfläche wird lediglich durch Durchgangsbohrungsöffnungen 31c und 31d der Durchgangsbohrung 31 unterbrochen. Sofern man einer solchen Unterbrechung im Hinblick auf eine Grundform überhaupt Bedeutung beimessen wollte, so kann die Außenfläche aber zumindest immer noch über Flächenabschnitte eines entsprechenden Ellipsoids beschrieben werden.

Die Längsachse L der Durchgangsbohrung verläuft hier zentrisch durch den ionenabgebenden Körper 3. In Bezug auf die Längsachse L wird hier die Länge der Halbachse c als Länge der Halbachse des ionenabgebenden Körpers 3 in Richtung der Längsachse L angegeben und entspricht im Sinne gleich langer Halbachsen a, b und c dem Kugelradius. Zur Orientierung ist in Figur 3a, oben rechts, auch nochmals ein entsprechendes Koordinatensystem angegeben. In einer Ausführungsvariante kann die Längsachse L der Durchgangsbohrung 31 aber auch exzentrisch durch den ionenabgebenden Körper 3 verlaufen.

Die Durchgangsbohrung 31 weist zudem in Richtung der Längsachse L zwei Durchgangsbohrungsabschnitte 31a und 31b mit unterschiedlichen Durchmessern d1 bezogen auf den Durchgangsbohrungsabschnitt 31a und d2 bezogen auf den Durchgangsbohrungsabschnitt 31b auf. Der Durchgangsbohrungsabschnitt 31b erstreckt sich ausgehend von der Durchgangsbohrungsöffnung 31d in Richtung der Längsachse L in den ionenabgebenden Körper 3 hinein, und der Durchgangsbohrungsabschnitt 31a erstreckt sich ausgehend von einem der Durchgangsbohrungsöffnung 31d abgewandten Ende des Durchgangsbohrungsabschnitts 31b in Richtung der Längsachse L bis zur Durchgangsbohrungsöffnung 31c. Der Durchmesser d1 des Durchgangsbohrungsabschnitts 31a ist kleiner als der Durchmesser d2 des Durchgangsbohrungsabschnitts 31b und derart dimensioniert, dass ein Knoten 4 des Fadens 5 den Durchgangsbohrungsabschnitt 31a nicht durchlaufen kann. Der größere Durchmesser d2 weist einen Durchmesser auf, der zur Aufnahme eines Knoten 4 des Fadens 5 geeignet ist. Demnach bildet der Übergang vom Durchgangsbohrungsabschnitt 31b mit dem größeren Durchmesser d2 zum Durchgangsbohrungsabschnitt 31a mit kleinerem Durchmesser d1 einen Anschlag für einen Knoten 4 des Fadens 5 aus, wobei ein Knoten 4 in der Anschlagsposition von dem Durchgangsbohrungsabschnitt 31b mit dem größeren Durchmesser d2 überdeckt wird. Die Durchmesser d1 und d2 sind hier in Richtung der Längsachse L konstant. In einer Ausgestaltungsvariante können die Durchgangsbohrungsabschnitte 31a und 31b aber auch veränderliche Durchmesser aufweisen, wobei sich der Durchmesser d1 bezogen auf den Durchgangsbohrungsabschnitt 31a auf einen minimalen Durchmesser und Durchmesser d2 bezogen auf den Durchgangsbohrungsabschnitt 31b auf einen maximalen Durchmesser bezieht. In einer weiteren Variante kann sich die Durchgangsbohrung 31 auch von der Durchgangsbohrungsöffnung 31d zur Durchgangsbohrungsöffnung 31c zumindest von einem minimalen Durchmesser d2 auf einen maximalen Durchmesser d1 verjüngen.

Die Anordnung des ionenabgebenden Körpers 3 in Bezug auf den Faden 5 der Vorrichtung 10 erfolgt derart, dass die Durchgangsbohrungsöffnung 31c als Öffnung des Durchgangsbohrungsabschnitts 31a mit kleinerem Durchmesser d1 nach außen auf einer dem Verankerungsknoten 1 zugewandten Seite des ionenabgebenden Körpers 3 liegt und die Durchgangsbohrungsöffnung 31d als Öffnung des Durchgangsbohrungsabschnitts 31b mit größerem Durchmesser d2 nach außen auf einer dem Verankerungsknoten 1 abgewandten Seite des ionenabgebenden Körpers 3 liegt.

Die Figuren 3b, 3c und 3d zeigen jeweils nochmals weitere Außenansichten des ionenabgebenden Körpers 3. Figur 3b stellt eine Seitenansicht, Figur 3c eine Ansicht von oben, also von außen auf die Durchgangsbohrungsöffnung 31c, und Figur 3d eine Ansicht von unten, also von außen auf die Durchgangsbohrungsöffnung 31d mit in dieser Blickrichtung dahinterliegender Durchgangsbohrungsöffnung 31c, dar.

Der Kugelradius liegt in der gezeigten Ausführungsform bei 1,5 mm, ist darauf aber nicht beschränkt. In Ausführungsvarianten kann der Kugelradius beispielsweise auch bei 2 mm, 2,5 mm, 3 mm, 3,5 mm, 4 mm, 4,5 mm oder 10 mm liegen. Weitere Größen oder auch Zwischengrößen sind denkbar.

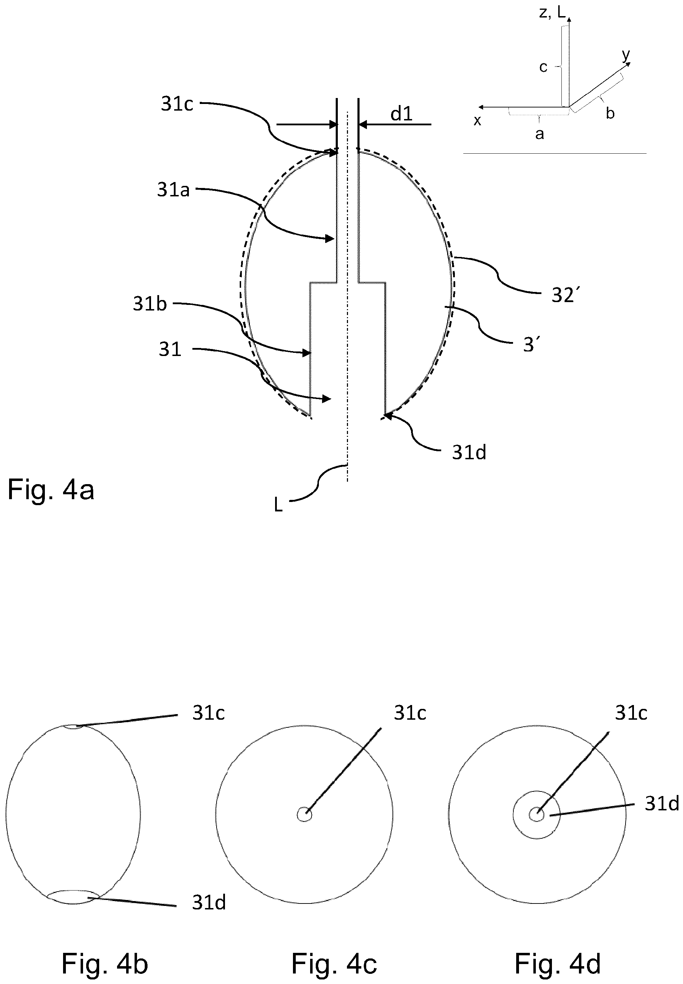

Figur 4a zeigt eine schematische Querschnittsansicht einer exemplarischen zweiten Ausführungsform eines ionenabgebenden Körpers 3' entlang der Längsachse L der Durchgangsbohrung 31. Die zweite Ausführungsform unterscheidet sich von der ersten Ausführungsform durch die Ausbildung der Außenfläche 32' des ionenabgebenden Körpers 3' als Rotationsellipsoid bzw. als Flächenabschnitt eines solchen Rotationsellipsoiden, sofern die über die Durchgangsbohrung 31 gebildeten Aussparungen als Abweichung in Betracht gezogen werden, wobei dies auf die Grundform keinen Einfluss hat.

Der ionenabgebende Körper 3' weist hier eine zur Längsachse L korrespondierende Rotationsachse im Hinblick auf die Rotationssymmetrie des ionenabgebenden Körpers 3' bzw. der Außenfläche 32' auf. Die Längen der Halbachsen a und b als Längen der Halbachsen senkrecht zur Rotationsachse bzw. Längsachse L sind demnach gleich. Demgegenüber weist die Länge der Halbachse c in Richtung der Längsachse L eine von den Längen der Halbachsen a und b unterschiedliche Länge auf. In Figur 4a ist die Länge der Halbachse c länger als die Längen der Halbachsen a und b. Gemäß einer Ausgestaltungsvariante kann die Länge der Halbachse c aber auch kürzer als die Längen der Halbachsen a und b sein.

In der gezeigten Ausführungsform betragen die Länge der Halbachse c exemplarisch 2 mm und die Längen der Halbachsen a und b jeweils 1,5 mm. Die Länge der Halbachse c kann aber beispielsweise 2,5 mm, 3 mm, 3,5 mm, 4 mm, 4,5 mm oder 5 mm betragen und die Länge der Halbachsen a und b kann jeweils gleichermaßen variieren.

Auch zur zweiten Ausführungsform zeigen die Figuren 4b, 4c und 4d jeweils nochmals weitere Außenansichten des ionenabgebenden Körpers 3'. Figur 4b stellt eine Seitenansicht, Figur 4c eine Ansicht von oben, also von außen auf die Durchgangsbohrungsöffnung 31c, und Figur 4d eine Ansicht von unten, also von außen auf die Durchgangsbohrungsöffnung 31d mit in dieser Blickrichtung dahinterliegender Durchgangsbohrungsöffnung 31c, dar.

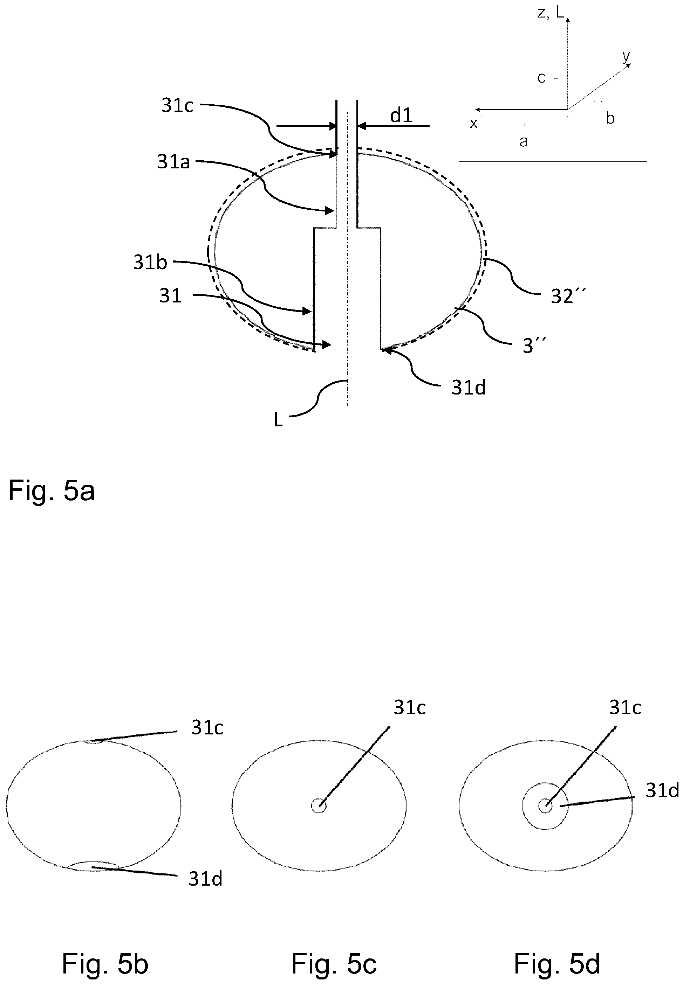

Figur 5a zeigt eine schematische Querschnittsansicht einer exemplarischen dritten Ausführungsform eines ionenabgebenden Körpers 3" entlang der Längsachse L der Durchgangsbohrung 31. Die dritte Ausführungsform unterscheidet sich von der zweiten Ausführungsform dadurch, dass der Rotationsellipsoid hier nicht um die Längsachse L als Symmetrieachse gebildet wird, sondern um eine zur Längsachse L senkrechte Achse, hier die Achse x als Erstreckungsrichtungsrichtung der Länge der Halbachse a. Mit anderen Worten sind hier die Längen der Halbachsen b und c gleich, wobei die Länge der Halbachse a hiervon abweicht und länger als die Länge der Halbachsen b und c ist.

Die Längen der Halbachsen b und c betragen hier jeweils 1, 5 mm und die Länge der Halbachse a liegt bei 2 mm. Auch hier können in anderen Ausführungsvarianten andere Längen der Halbachsen a, b und c vorgesehen werden. Beispielsweise kann die Länge der Halbachse a auch bei 2,5 mm oder 3 mm liegen.

Auch zur dritten Ausführungsform zeigen die Figuren 5b, 5c und 5d jeweils nochmals weitere Außenansichten des ionenabgebenden Körpers 3". Figur 5b stellt eine Seitenansicht, Figur 5c eine Ansicht von oben, also von außen auf die Durchgangsbohrungsöffnung 31c, und Figur 5d eine Ansicht von unten, also von außen auf die Durchgangsbohrungsöffnung 31d mit in dieser Blickrichtung dahinterliegender Durchgangsbohrungsöffnung 31c, dar.

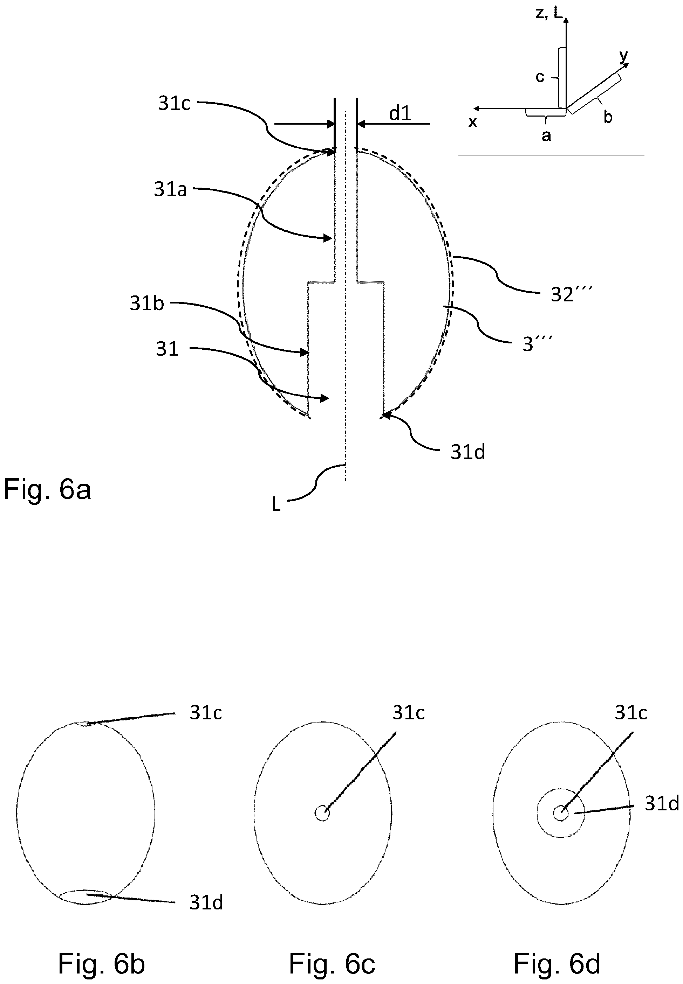

Figur 6a zeigt eine schematische Querschnittsansicht einer exemplarischen vierten Ausführungsform eines ionenabgebenden Körpers 3‴ entlang der Längsachse L der Durchgangsbohrung 31. Die vierte Ausführungsform unterscheidet sich von der dritten Ausführungsform dadurch, dass hier die Länge der Halbachse a gegenüber den gleich langen Halbachsen b und c verkürzt ist.

Die Längen der Halbachsen b und c betragen hier jeweils 2 mm und die Länge der Halbachse a liegt bei 1,5 mm. Auch hier können in anderen Ausführungsvarianten andere Längen der Halbachsen a, b und c vorgesehen werden. Beispielsweise können die Längen der Halbachsen b und d auch bei 2,5 mm, 3 mm, 3,5 mm, 4 mm, 4,5 mm oder 5 mm liegen.

Auch zur vierten Ausführungsform zeigen die Figuren 6b, 6c und 6d jeweils nochmals weitere Außenansichten des ionenabgebenden Körpers 3". Figur 6b stellt eine Seitenansicht, Figur 6c eine Ansicht von oben, also von außen auf die Durchgangsbohrungsöffnung 31c, und Figur 6d eine Ansicht von unten, also von außen auf die Durchgangsbohrungsöffnung 31d mit in dieser Blickrichtung dahinterliegender Durchgangsbohrungsöffnung 31c, dar.

Die ionenabgebenden Körper 3, 3', 3" und 3‴ der ersten bis vierten Ausführungsform sind jeweils aus Kupfer. In alternativen Ausgestaltungsvarianten können die ionenabgebenden Körper 3, 3', 3" und 3‴ aber auch aus einer Kupferlegierung, insbesondere aus einer Kupfer-Gold-Legierung, oder aus einer Gold oder Goldlegierung, insbesondere einer Gold-Kupfer-Legierung, gebildet werden oder mit einem entsprechend vorstehenden Material zumindest teilweise außen beschichtet sein.

Die Erfindung ist nicht auf die beschriebenen Ausführungsformen beschränkt. Auch wenn in den vorstehend beschriebenen Ausführungsformen lediglich jeweils ionenabgebende Körper gezeigt sind, die gemäß ihrer Außenfläche in ihrer Grundform ein Ellipsoid nachbilden, kann die Außenfläche der ionenabgebenden Körper auch lediglich Flächenabschnitte eines Ellipsoids aufweisen und im Übrigen hiervon abweichende Flächenbereiche ausbilden, die aber in Übergängen keine vorstehenden Kanten aufweisen. Ebenso kann die Außenfläche des ionenabgebenden Körpers aus mehreren Flächenabschnitten eines Ellipsoids zusammengesetzt werden. Eine weitere Ausgestaltungsvariante besteht in einer Oberflächenstrukturierung, beispielsweise über konkave oder konvexe Ausbuchtungen.

Bezugszeichenliste

| | |
|---|---|
| 1 | Verankerungsknoten (Vorrichtungsfixierung) |
| 2 | Edelstahlclip |
| 3, 3', 3", 3‴ | ionenabgebender Körper |
| 4 | Knoten (Abstandshalter) |
| 5 | Faden |
| 31 | Durchgangsbohrung |
| 31a | Durchgangsbohrungsabschnitt (d1) |
| 31b | Durchgangsbohrungsabschnitt (d2) |
| 31c | Durchgangsbohrungsöffnung (d1) |
| 31d | Durchgangsbohrungsöffnung (d2) |
| 32, 32', 32", 32‴ | Außenfläche |
| a, b, c | Halbachsen |
| d1, d2 | Durchmesser |
| L | Längsachse |
| x, y, z | kartesische Koordinaten |

**Patentansprüche**

1. Ionenabgebender Körper (3, 3', 3", 3‴) für eine Vorrichtung (10) zur Empfängnisverhütung, aufweisend:

   zumindest eine Durchgangsbohrung (31) mit zumindest zwei Durchgangsbohrungsöffnungen (31c, 31d), wobei eine sich von einer der Durchgangsbohrungsöffnungen (31c, 31d) zur anderen der Durchgangsbohrungsöffnungen (31d, 31c) erstreckende Außenfläche (32, 32', 32", 32‴) des ionenabgebenden Körpers (3, 3', 3", 3‴) keine nach außen weisende Kante aufweist, **dadurch gekennzeichnet, dass** die Durchgangsbohrung (31) zumindest zwei unterschiedliche Durchmesser (d1, d2) aufweist.

2. Vorrichtung (10) zur Empfängnisverhütung aufweisend:

   zumindest einen Faden (5) sowie zumindest einen ionenabgebenden Körper (3, 3', 3", 3‴) nach Anspruch 1, wobei der zumindest eine Faden (5) durch die zumindest zwei Durchgangsbohrungsöffnungen (31c, 31d) verläuft.

3. Vorrichtung (10) zur Empfängnisverhütung nach Anspruch 2, wobei die Außenfläche (32, 32', 32", 32‴) des ionenabgebenden Körpers (3, 3', 3", 3‴) zumindest abschnittsweise als Flächenabschnitt eines Ellipsoids mit a, b und c als Längen der Halbachsen eines solchen Ellipsoids, insbesondere in kartesischen Koordinaten über die Gleichung

$$\frac{x^2}{a^2} + \frac{y^2}{b^2} + \frac{z^2}{c^2} = 1$$

beschreibbar ist, wobei a, b und c größer null sind.

4. Vorrichtung (10) zur Empfängnisverhütung nach Anspruch 3, wobei sich eine Längsachse (L) des ionenabgebenden Körpers (3, 3', 3", 3‴) im Wesentlichen parallel zu einer Erstreckungsrichtung der Durchgangsbohrung (31) des ionenabgebenden Körpers (3, 3', 3", 3‴) erstreckt und wobei sich die Längen der Halbachsen a, b senkrecht zur Längsachse (L) erstrecken und sich die Länge der Halbachse c in Richtung der Längsachse (L) erstreckt.

5. Vorrichtung (10) zur Empfängnisverhütung nach Anspruch 3 oder 4, wobei die Längen der Halbachsen a, b und c gleich sind.

6. Vorrichtung (10) zur Empfängnisverhütung nach Anspruch 5, wobei die Längen der Halbachsen a, b und c zwischen 1 mm und 5,5 mm, insbesondere zwischen 1,5 mm und 5 mm, liegen.

7. Vorrichtung (10) zur Empfängnisverhütung nach Anspruch 3 oder 4, wobei zwei der Längen der Halbachsen a, b und c übereinstimmen und eine der Längen der Halbachsen a, b und c hiervon abweicht.

8. Vorrichtung (10) zur Empfängnisverhütung nach Anspruch 7, wobei die Längen der Halbachsen a und b gleich sind.

9. Vorrichtung (10) zur Empfängnisverhütung nach Anspruch 8, wobei die Längen der Halbachsen a und b zwischen 1 mm und 2 mm liegen, insbesondere in etwa 1,5 mm betragen, und die Länge der Halbachse c zwischen 1,5 mm und 5,5 mm, insbesondere zwischen 2 mm und 5 mm, liegt.

**10.** Vorrichtung (10) zur Empfängnisverhütung nach Anspruch 7, wobei die Längen der Halbachsen a und c oder b und c gleich sind.

**11.** Vorrichtung (10) zur Empfängnisverhütung nach Anspruch 10, wobei die Länge der Halbachse c zwischen 1 mm und 5,5 mm, insbesondere zwischen 1,5 mm und 5 mm, liegt und die hiervon unterschiedliche Länge der Halbachse a oder b zwischen 1 mm und 3,5 mm, insbesondere zwischen 1,5 mm und 3 mm, liegt.

**12.** Vorrichtung (10) zur Empfängnisverhütung nach Anspruch 3 oder 4, wobei die Längen der Halbachsen a, b und c jeweils zueinander unterschiedlich sind.

**13.** Vorrichtung (10) zur Empfängnisverhütung nach einem der vorherigen Ansprüche, wobei der ionenabgebende Körper (3, 3', 3", 3‴) ein Metall, insbesondre Kupfer, eine Kupferlegierung, Gold, eine Goldlegierung oder eine Kupfer-Gold-Legierung, enthält oder hieraus gebildet wird.

**14.** Vorrichtung (10) zur Empfängnisverhütung nach einem der vorherigen Ansprüche, wobei die Vorrichtung 2 bis 5 ionenabgebende Körper (3, 3', 3", 3‴), insbesondere 3 oder 4 ionenabgebende Körper (3, 3', 3", 3‴), aufweist, durch deren jeweilige Durchgangsbohrungen (31) der zumindest eine Faden (5) verläuft.

**Claims**

**1.** Ion-emitting body (3, 3', 3", 3‴) for a device (10) for contraception, comprising:

> at least one through hole (31) with at least two through hole openings (31c, 31d) wherein an outer surface (32, 32', 32", 32‴) of the ion-emitting body (3, 3', 3", 3‴) extending from one of the through hole openings (31c, 31d) to the other one of the through hole openings (31d, 31c) has no outwardly facing edge, **characterized in that** the through hole (31) has at least two different diameters (d1, d2).

**2.** Device (10) for contraception, comprising:

> at least one thread (5) and at least one ion-emitting body (3, 3', 3", 3‴) according to claim 1, wherein the at least one thread (5) extends through the at least two through hole openings (31c, 31d).

**3.** Device (10) for contraception according to claim 2, wherein the outer surface (32, 32', 32", 32‴) of the ion-emitting body (3, 3', 3", 3‴) is at least in sections describable as a surface section of an ellipsoid with a, b and c as lengths of the semi-axes of such an ellipsoid, in particular in Cartesian coordinates via the equation

$$\frac{x^2}{a^2} + \frac{y^2}{b^2} + \frac{z^2}{c^2} = 1$$

wherein a, b and c are greater than zero.

**4.** Device (10) for contraception according to claim 3, wherein a longitudinal axis (L) of the ion-emitting body (3, 3', 3", 3‴) extends substantially parallel to an extension direction of the through hole (31) of the ion-emitting body (3, 3', 3", 3‴), and wherein the lengths of the semi-axes a, b extend perpendicular to the longitudinal axis (L) and the length of the semi-axis c extends in the direction of the longitudinal axis (L).

**5.** Device (10) for contraception according to claim 3 or 4, wherein the lengths of the semi-axes a, b and c are equal.

**6.** Device (10) for contraception according to claim 5, wherein the lengths of the semi-axes a, b and c are between 1 mm and 5.5 mm, in particular between 1.5 mm and 5 mm.

**7.** Device (10) for contraception according to claim 3 or 4, wherein two of the lengths of the semi-axes a, b and c are equal and one of the lengths of the semi-axes a, b and c is different therefrom.

**8.** Device (10) for contraception according to claim 7, wherein the lengths of the semi-axes a and b are equal.

**9.** Device (10) for contraception according to claim 8, wherein the lengths of the semi-axes a and b are between 1 mm and 2 mm, in particular approximately 1.5 mm, and the length of the semi-axis c is between 1.5 mm and 5.5 mm, in particular between 2 mm and 5 mm.

**10.** Device (10) for contraception according to claim 7, wherein the lengths of the semi-axes a and c, or b and c are equal.

**11.** Device (10) for contraception according to claim 10, wherein the length of the semi-axis c is between 1 mm and 5.5 mm, in particular between 1.5 mm and 5 mm, and the length of the semi-axis a or b different therefrom is between 1 mm and 3.5 mm, in particular between 1.5 mm and 3 mm.

**12.** Device (10) for contraception according to claim 3 or 4, wherein the lengths of the semi-axes a, b and c are each different from one another.

**13.** Device (10) according to any one of the preceding claims, wherein the ion-emitting body (3, 3', 3", 3‴) contains or is formed from a metal, in particular copper, a copper alloy, gold, a gold alloy or a copper-gold alloy.

**14.** Device (10) for contraception according to any one of the preceding claims, wherein the device comprises 2 to 5, in particular 3 or 4, ion-emitting bodies (3, 3', 3", 3‴) through the respective through holes (31) of which the at least one thread (5) extends.

**Revendications**

**1.** Corps émetteur d'ions (3, 3', 3", 3‴) pour un dispositif (10) de contraception, comprenant:

au moins un trou traversant (31) ayant au moins deux ouvertures de trou traversant (31c, 31d), dans lequel une surface extérieure (32, 32', 32", 32‴) du corps émetteur d'ions (3, 3', 3", 3‴) s'étendant de l'une des ouvertures de trou traversant (31c, 31d) à l'autre des ouvertures de trou traversant (31d, 31c) n'a pas de bord tourné vers l'extérieur, **caractérisé en ce que** le trou traversant (31) a au moins deux diamètres différents (d1, d2).

**2.** Dispositif (10) de contraception, comprenant :

au moins un fil (5) et
au moins un corps émetteur d'ions (3, 3', 3", 3‴) selon la revendication 1,
dans lequel le au moins un fil (5) s'étend à travers les au moins deux ouvertures de trou traversant (31c, 31d).

**3.** Dispositif (10) de contraception selon la revendication 2, dans lequel la surface extérieure (32, 32', 32", 32‴) du corps émetteur d'ions (3, 3', 3", 3‴) peut être décrite au moins par sections comme une section de surface d'un ellipsoïde ayant a, b et c comme longueurs des demi-axes d'un tel ellipsoïde, en particulier en coordonnées cartésiennes par l'équation

$$\frac{x^2}{a^2} + \frac{y^2}{b^2} + \frac{z^2}{c^2} = 1$$

dans lequel a, b et c sont supérieurs à zéro.

**4.** Dispositif (10) de contraception selon la revendication 3, dans lequel un axe longitudinal (L) du corps émetteur d'ions (3, 3', 3", 3‴) s'étend sensiblement parallèlement à une direction d'extension du trou traversant (31) du corps émetteur d'ions (3, 3', 3", 3‴), et dans lequel les longueurs des demi-axes a, b s'étendent perpendiculairement à l'axe longitudinal (L) et la longueur du demi-axe c s'étend dans la direction de l'axe longitudinal (L).

**5.** Dispositif (10) de contraception selon la revendication 3 ou 4, dans lequel les longueurs des demi-axes a, b et c sont égales.

**6.** Dispositif (10) de contraception selon la revendication 5, dans lequel les longueurs des demi-axes a, b et c sont comprises entre 1 mm et 5,5 mm, en particulier entre 1,5 mm et 5 mm.

**7.** Dispositif (10) de contraception selon la revendication 3 ou 4, dans lequel deux des longueurs des demi-axes a, b et c sont égales et l'une des longueurs des demi-axes a, b et c est différente de celle-ci.

**8.** Dispositif (10) de contraception selon la revendication 7, dans lequel les longueurs des demi-axes a et b sont égales.

**9.** Dispositif (10) de contraception selon la revendication 8, dans lequel les longueurs des demi-axes a et b sont comprises entre 1 mm et 2 mm, en particulier d'environ 1,5 mm, et la longueur du demi-axe c est comprise entre 1,5 mm et 5,5 mm, en particulier entre 2 mm et 5 mm.

**10.** Dispositif (10) de contraception selon la revendication 7, dans lequel les longueurs des demi-axes a et c, ou b et c sont égales.

**11.** Dispositif (10) de contraception selon la revendication 10, dans lequel la longueur du demi-axe c est comprise entre 1 mm et 5,5 mm, en particulier entre 1,5 mm et 5 mm, et la longueur du demi-axe a ou b différent de celle-ci est comprise entre 1 mm et 3,5 mm, en particulier entre 1,5 mm et 3 mm.

**12.** Dispositif (10) de contraception selon la revendication 3 ou 4, dans lequel les longueurs des demi-axes a, b et c sont différentes les unes des autres.

**13.** Dispositif (10) selon l'une quelconque des revendications précédentes, dans lequel le corps émetteur d'ions (3, 3', 3", 3‴) contient ou est formé d'un métal, en particulier cuivre, un alliage de cuivre, or, un alliage d'or ou un alliage de cuivre-or.

**14.** Dispositif (10) de contraception selon l'une quelconque des revendications précédentes, dans lequel le dispositif comprend 2 à 5, en particulier 3 ou 4, corps émetteurs d'ions (3, 3', 3", 3‴) à travers les trous traversants (31) respectifs desquels le au moins un fil (5) s'étend.

**Fig. 1**

**Fig. 2**

Fig. 3a

Fig. 3b          Fig. 3c          Fig. 3d

31c

31a

31b

31

d1

z, L

c

x

y

b

a

32′

3′

31d

L

Fig. 4a

31c

31c

31c

31d

31d

Fig. 4b

Fig. 4c

Fig. 4d

Fig. 5a

Fig. 5b          Fig. 5c          Fig. 5d

31c

31a

31b

31

d1

z, L

c

x

y

b

a

32'''

3'''

31d

**Fig. 6a**

L

31c

31c

31c

31d

31d

**Fig. 6b**

**Fig. 6c**

**Fig. 6d**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2009155367 A1
- US 2018235803 A1
- US 2017246027 A1
- EP 0673629 A1
- EP 2308428 A1
- US 4949732 A